# EUROPEAN PATENT SPECIFICATION

(11) **EP 1 957 632 B1**
(45) Date of publication and mention of the grant of the patent: **30.05.2012**
(21) Application number: 06807548.0
(22) Date of filing: 25.10.2006
(51) Int. Cl.: C12N 5/07

(54) **TISSUE ENGINEERING USING PURE POPULATIONS OF ISOLATED NON-EMBRYOBLASTIC FETAL CELLS**
GEWEBEREKONSTRUKTION UNTER VERWENDUNG REINER POPULATIONEN ISOLIERTER FÖTALER NICHTEMBRYOBLASTENZELLEN
PRODUCTION DE TISSUS PAR INGENIERIE GENETIQUE A L'AIDE DE POPULATIONS PURES DE CELLULES FOETALES ISOLEES NON EMBRYOBLASTIQUES

(30) Priority: 28.10.2005 EP 05023702
(43) Date of publication of application: 20.08.2008
(60) Divisional application: 10179831.2
(73) Proprietor: Universität Zürich, 8006 Zürich (CH)
(72) Inventor: SCHMIDT, Dörthe, CH-8008 Zürich (CH); BREYMANN, Christian, 8702 Zollikon (CH); ZÜND, Gregor, CH-8704 Herrliberg (CH); HOERSTRUP, Simon P., CH-8032 Zürich (CH); ACHERMANN, Josef, CH-8032 Zürich (CH)
(74) Representative: Bremi, Tobias Hans
(86) International application number: PCT/EP2006/067775
(87) International publication number: WO 2007/048813

(56) References cited:
- WO-A2-03/042405
- PRUSA ANDREA-ROMANA ET AL: "Amniotic fluid cells and human stem cell research: a new connection." MEDICAL SCIENCE MONITOR : INTERNATIONAL MEDICAL JOURNAL OF EXPERIMENTAL AND CLINICAL RESEARCH NOV 2002, vol. 8, no. 11, November 2002 (2002-11), pages RA253-RA257, XP002419092 ISSN: 1234-1010
- KAVIANI AMIR ET AL: "The placenta as a cell source in fetal tissue engineering." JOURNAL OF PEDIATRIC SURGERY JUL 2002, vol. 37, no. 7, July 2002 (2002-07), pages 995-999 ; dis, XP002419093 ISSN: 1531-5037
- DE COPPI PAOLO ET AL: "Pluripotent stem cells derived from human chorionic villi and amniotic fluid for tissue engineering applications" FASEB JOURNAL, vol. 19, no. 5, Suppl. S, Part 2, March 2005 (2005-03), page A1366, XP009078624 & EXPERIMENTAL BIOLOGY 2005 MEETING/35TH INTERNATIONAL CONGRESS OF PHYSIOLOGICAL SCIENCES; SAN DIEGO, CA, USA; MARCH 31 -APRIL 06, 2005 ISSN: 0892-6638
- UNGER R E ET AL: "Endothelialization of a non-woven silk fibroin net for use in tissue engineering: growth and gene regulation of human endothelial cells" BIOMATERIALS, ELSEVIER SCIENCE PUBLISHERS BV., BARKING, GB, vol. 25, no. 21, September 2004 (2004-09), pages 5137-5146, XP004504203 ISSN: 0142-9612
- HOERSTRUP SIMON P ET AL: "Tissue engineering of functional trileaflet heart valves from human marrow stromal cells" CIRCULATION, vol. 106, no. 13 Supplement, 24 September 2002 (2002-09-24), pages I-143, XP002419094 ISSN: 0009-7322
- SCHMIDT D ET AL: "Umbilical Cord Blood Derived Endothelial Progenitor Cells for Tissue Engineering of Vascular Grafts" THE ANNALS OF THORACIC SURGERY, ELSEVIER, vol. 78, no. 6, December 2004 (2004-12), pages 2094-2098, XP004700032 ISSN: 0003-4975
- DELLA ROCCA F ET AL: "Cell composition of the human pulmonary valve: a comparative study with the aortic valve--the VESALIO Project. Vitalitate Exornatum Succedaneum Aorticum labore Ingegnoso Obtinebitur." THE ANNALS OF THORACIC SURGERY NOV 2000, vol. 70, no. 5, November 2000 (2000-11), pages 1594-1600, XP002419095 ISSN: 0003-4975
- SCHMIDT DOERTHE ET AL: "Tissue engineered heart valves based on human cells" SWISS MEDICAL WEEKLY, vol. 136, no. 39-40, September 2006 (2006-09), pages 618-623, XP002419096 ISSN: 1424-7860
- SCHMIDT DOERTHE ET AL: "Living autologous heart valves engineered from human prenatally harvested progenitors" CIRCULATION, vol. 114, no. Suppl. 1, July 2006 (2006-07), pages I125-I131, XP002419097 ISSN: 0009-7322

## Description

The present invention relates to methods for the *in vitro* production of mammalian tissue replacements using substantially pure populations of isolated non-embryoblastic fetal cells having the capacity to differentiate into the cell type(s) that form(s) the native tissue. The tissue replacements engineered by the methods of the present invention are especially useful for the repair of non-functional or malfunctional cardiovascular structures in patients suffering from congenital cardiovascular disorders.

The progress in the treatment and regeneration of congenital malformation and defects has been significant in the past decades, providing a lifesaving surgical treatment for numerous patients each year. Today, for example, cardiovascular replacements by either mechanical or biological prostheses remain the most common treatment for advanced valvular heart disease with an increasing use of biological prostheses. However, this therapy is still associated with a number of problems resulting in a significant morbidity and mortality. Being inherently different from the tissue they replace, both manufactured mechanical and biological valve replacements are often associated with shortcomings such as material failure, increased rate of infections, thromboembolism and immunological reactions against the foreign material. In addition, with the exception of the Ross Principle, all contemporary cardiovascular replacement procedures involve non-living structures lacking the capacity for self-repair, remodeling or growth.

Particularly in today's congenital heart surgery, there is a substantial need for appropriate, growing replacement materials for the repair of congenital cardiac defects. This surgical treatment is commonly based on non-autologous valves or conduits with disadvantages including obstructive tissue ingrowths and calcification of the replacement. These limitations and the lack of growth typically necessitate various re-operations of the pediatric patients with cardiovascular defects associated with increased morbidity and mortality each time.

Ideal tissue replacements would be a copy of their native counterparts. Particularly in cardiovascular tissue engineering such replacements should exhibit adequate mechanical function, durability, adequate haemodynamic performance, as well as the absence of immunogenic, thrombogenic and/or inflammatory reactions.

Tissue engineering aims to match these requirements by *in vitro* fabrication of living, autologous tissue replacements. Therefore, autologous cells are obtained and isolated from the patient's tissue. After isolation the cells are expanded using *in vitro* culturing technology and seeded onto biodegradable three-dimensional matrices, which can be of biological or synthetic origin. For the seeding procedure a sufficient initial number of cells are necessary in order to enable appropriate maturation of the neo-tissue. The success of this tissue engineering procedure depends on three main elements: (1) the biodegradable matrix (scaffold) which determines the three-dimensional shape and serves as an initial guiding structure for cell attachment and tissue development; (2) the cell source from which a living tissue is grown; and (3) the *in vitro* culture conditions of the living construct before implantation.

Particularly in cardiovascular tissue engineering, these three elements have to be chosen and controlled in a highly orchestrated manner to meet the high mechanical requirements of the neo-tissue at the time of implantation. For example, in order to create a functional heart valve with the mechanical properties of the native counterpart, a rapid development of the extracellular matrix is crucial. Therefore, the choice of cells which are responsible for the production of an extracellular matrix is an important factor. Two cell types are currently used for the fabrication of cardiovascular tissues: cells with the capacity to form extracellular matrix, commonly myofibroblasts, and endothelial cells with antithrombogenic characteristics. The seeding procedure onto three-dimensional scaffolds is mostly performed sequentially: first by seeding of the myofibroblasts, followed by the endothelial cells (Zund et al. (1998) Eur. J. Cardiothorac. Surg. 13, 160-164). The seeded scaffolds can be cultured either in static or dynamic systems, aiming at optimal tissue development *in vitro.* It has been shown that mechanical preconditioning accelerates the production of viable, functional tissues making them appropriate for implantation (Niklason et al. (1999) Science 284, 489-493; Hoerstrup et al. (2000) Tissue Eng. 6, 75-79).

In contrast to the highly standardized and industrially fabricated scaffolds the quality of cells varies from patient to patient, depending on the individual tissue characteristics and co-morbidities. In order to create a functional, living tissue replacement the choice of the cell source is critical. Besides cell growth and expansion capacity, an important issue is the possibility to develop a cell phenotype that matches the native counterparts. This is expected to have a major impact on the long-term functionality of the replacements (Butcher et al. (2004) J. Heart Valve Disease 13, 478-486). Using cells originating from the tissue to be replaced would be the safest approach. In the case of heart valve tissue engineering, the usage of valvular interstitial cells obtained by biopsy has been shown feasible (Maish et al. (2003) J. Heart Valve Disease 12, 264-269). However, with respect to clinical applications, these cells are difficult to obtain and the approach bears substantial risks.

Particularly for the fabrication of tissue engineered replacements for pediatric applications, the ideal cell source has not been identified yet. The ideal cell source should be easily accessible and must allow a prenatal cell harvesting in order to have the tissue-engineered construct ready at or shortly after birth preventing secondary damage of the infant heart.

Previous studies described prenatal ewes cells from amniotic fluid as a new cell source for tissue engineering for diaphragma reconstruction in an animal model (Kaviani et al. (2003) J. Am. Coll. Surg. 196, 592-597; Fuchs et al. (2004) J. Ped. Surg. 39, 834-838). Kaviani et al. (J. Ped. Surg. 37, 995-999, 2002) reported the use of amniotic fluid derived cells obtained at 16 to 21 weeks compared to postnatal human placental cells obtained from cesarean section-delivered placenta at 33 to 35 week of gestation. However, so far neither tissue formation including connective extracellular matrix elements nor functionality of constructs with complex geometry grown from these cells could be demonstrated.

Accordingly, the technical problem underlying the present invention is to provide improved methods for the production of mammalian tissue replacements.

The solution to the above technical problem is provided by the embodiments of the present invention as defined in the claims.

In particular, according to a first aspect, the present invention provides a method for the *in vitro* production of a mammalian tissue placement comprising the steps of:
(a) preparing one or more substantially pure population(s) of isolated non-embryoblastic fetal cells of one or more type(s) *in vitro,* wherein the cell type(s) has/have the capacity to form the native tissue corresponding to the replacement; and
(b) cultivating the fetal cells obtained in step (a) under conditions allowing the development of the tissue replacement.

The present invention is based at least in part on the finding that the separation and isolation of those non-embryoblastic fetal cell types that have the capacity of forming the desired tissue replacement is essential for the development of replacements that most closely resemble their corresponding structures developed in the natural surrounding, thus exhibiting optimal biochemical, mechanical and physiological properties.

The fetal cells used in the method according to the present invention may thus be selected according to the cell type(s) present in the naturally occurring tissue that is to be replaced.

Examples of suitable fetal cells are fibroblasts, myofibroblasts, hematopoietic cells, endothelial cells, chondrocytes, chondroblasts, osteocytes, osteoblasts, epithelial cells as well progenitors of such cell types.

In order to provide tissue replacements that correspond in their characteristics as good as possible to the native counterparts, it is essential that the fetal cells are isolated in a manner that substantially pure cell types are separated from one another. The expression "substantially pure cell population", as used herein, means a homologous population of cells displaying not only the morphological but also the functional properties of the respective cell type or lineage. Therefore, according to the present invention, the substantially homologous cell population contains, e.g. not more than 5%, preferably not more than 1%, more preferably not more than 0.1 % of cells not belonging to the respective desired cell type. In other words, the population of isolated cells is, e.g. at least 95%, preferably at least 99%, more preferably at least 99.9 % pure.

As known by a person skilled in the art, the desired cell type(s) may conveniently be identified and, according to preferred embodiments of the present invention, isolated and separated by the use of molecular markers such as intracellular or cell surface markers that are characteristic for the individual cell type(s). For example, the desired fetal cells may be isolated by appropriate cell sorting techniques, preferably flow cytometric methods, in particular fluorescence-activated cell sorting (FACS) and/or magnetic cell sorting, using antibodies directed against cell type-specific antigens, especially cell surface antigens, such as CD133, CD34 or other specific markers. Antibodies against cell type-specific antigens, optionally labelled with appropriate fluorescence tags or coupled to magnetic beads, are commercially available from various suppliers, e.g. Serotec Ltd., Oxford, UK. Information about which antigen should be selected in order to isolate a particular cell type is also available from the suppliers of corresponding antibodies. The sorting procedure according to the present inventions allows to separate different cell types and to cultivate, if necessary, two or more cell types which are necessary to form a tissue replacement in a highly orchestrated manner such that the replacement optimally fulfils the mechanical, physiological and biochemical requirements of the native tissue.

Equipment for cell sorting, in particular FACS or magnetic cell sorting, is commercially available, e.g. FACS equipment may be obtained from Becton Dickinson, Franklin Lakes, NJ, USA (FACStar^{®} Plus). Components and devices for magnetic cell sorting are available, e.g. MACS^{®} from Miltenyi Biotec GmbH, Bergisch-Gladbach, Germany. According to further preferred embodiments of the present invention, the isolation and separation of the desired cell type(s) is carried out using commercially available automated high-throughput systems (HTS). FACS as well as magnetic cell sorting is especially suitable for this purpose.

The term "mammalian tissue replacement" according to the present invention means any tissue present in a mammalian species that needs to be replaced due to a dysfunction or malfunction. Examples of tissue replacements engineered by the method according to the present invention include cardiovascular structures such as heart valves and parts thereof, blood vessels and parts thereof (e.g. patches), diaphragma replacements, cartilage, bone tissue, dermal replacements and so on. In principle, any tissue may be engineered by the methods of the present invention by choosing the required cell type(s) or their progenitors necessary to form the desired tissue (e.g. chondrocytes and/or chondroblasts or their progenitors for engineering cartilage, osteoblasts and/or osteocytes or their progenitors for producing bone tissue, epithelial cells or their progenitors for the fabrication of dermal replacements etc.). Preferably, progenitor cells are chosen, isolated, differentiated into the desired cell type(s) using appropriate growth factors, expanded and seeded onto a suitable scaffold.

In the case of tissue replacements such as cardiovascular structures (e.g. heart valves, blood vessels or parts thereof such as patches) or diaphragmatic reconstruction structures step (b) of the above-defined method comprises the sub-steps of:
(i) seeding the fetal cells obtained in the above-defined step (a) onto a three-dimensional scaffold; and
(ii) cultivating the scaffold under conditions allowing the development of the tissue replacement.

A "three-dimensional scaffold", as used herein, means a carrier for the cultivation of the fetal cells such that these build a functional tissue that can replace a naturally-occurring counterpart. The scaffold or carrier is an acellular structure, preferably built up of synthetic fibres or an acellular connective tissue matrix. Therefore, the material forming the three-dimensional scaffold is preferably a structure containing polymeric fibres, a porous polymer structure or an acellular biological tissue matrix. Typically, the scaffold is biologically degradable such that, when implanted into a patient in need of the corresponding tissue replacement, the scaffold is degraded after a certain period of time leaving the remaining mature tissue replacement which has been formed by the isolated non-embryoblastic fetal cells. Examples of biologically degradable carrier materials are polyglycolic acids (PGA), polylactic acid (PLA), polyhydroxyalcanoate (PHA) and poly-4-hydroxybutyrate (P4HB) and mixtures of two or more of the above materials as well as mixtures with one or more other suitable polymer(s). PHA and especially PH4B are particularly preferred, since these materials are thermo-mouldable due to their thermal plasticity such that they may be moulded into any desired shape, e.g. into a heart valve, conduit or part thereof. As mentioned before, the above polymers may be used alone or as mixtures of two or more of the above mentioned substances as well as mixtures of the substances together with other biologically degradable polymers. According to a preferred embodiment of the present invention the three-dimensional scaffold is a polymer mixture containing 85% PGA and 15% PLA. According to another preferred embodiment, the three-dimensional scaffold is made of a polymer blend containing PGA and P4HB (optionally together with other components) in amounts ranging from about 50 to about 99 % PGA and an appropriate amount of P4HB such as 20 to about 0.1 % P4HB. Particularly preferred blends are mixtures of 90% PGA and 10% P4HB or 99% PGA and 1% P4HB. Such polymer blends are typically mouldable at temperatures of about 60 to 70°C which enables that they may be formed into tubular structures (e.g. in order to build vessels or parts thereof) or heart valves.

Further preferred embodiments with respect to suitable scaffolds and their desired properties, in particular with respect to cardiovascular tissue replacements, are disclosed in EP-A-1 077 072.

Especially in the case of cardiovascular replacements, the above preferred embodiment of the method according to the present invention wherein the fetal cells are seeded onto a three-dimensional scaffold which is then cultivated under conditions allowing the development of the desired tissue replacement may be carried out by using different cell types which are preferably seeded and cultivated in a sequential manner. Therefore, according to a further preferred embodiment, the above method using three-dimensional scaffolds comprises the sub-steps of:
(1) seeding fetal cells having an extracellular matrix-forming capacity onto a three-dimensional scaffold;
(2) cultivating the scaffold until a connective tissue structure has been formed;
(3) seeding fetal cells having antithrombogenic characteristics onto the scaffold containing the connective tissue structure and
(4) further cultivating the scaffold onto at least a monolayer of the fetal cells having antithrombogenic properties has been formed on the connective tissue structure.

Therefore, in particular for the production of cardiovascular structures, it is essential to provide the three-dimensional scaffolds not only with cells forming an extracellular matrix containing its typical components, e.g. collagen, elastine and glycosaminoglycanes (besides the cells that build up the basic extracellular components), but also to provide the thus formed connective tissue structure with a cell layer having antithrombogenic characteristics. Typically, the cells having an extracellular matrix-forming capacity are fibroblasts and/or myofibroblasts or their progenitor cells. Furthermore, fetal cells having antithrombogenic properties may be selected from endothelial cells or progenitor cells thereof.

With respect to further preferred embodiments of this preferred method according of the present invention it is again referred to the respective disclosure content of EP-A-1 077 072.

According to preferred embodiments of the present invention the source of the non-embryoblastic fetal cells may be maternal blood, maternal tissue, amniotic fluid and/or chorionic villi.

Concerning the anatomic structures and tissues of the desired replacements, the time point of cell harvesting is an important factor with respect to accessible cell types and cell quality. Particularly in the non-embryoblastic fetal part of the donor, the development from secondary chorionic villi to mesenchymal tertiary chorionic villi of vascular origin is an important time-depending reconstruction (starting at around the 3^{rd} week of pregnancy). These villi represent an attractive cell source of various different progenitor cells until they are again transformed into mature intermediate villi, rather than into immature ones by approximately the 23^{rd} week of pregnancy. Therefore, in general, but in particular with respect to cells obtained from chorionic villi, harvesting the desired cells at an early stage of pregnancy, e.g. in the range of from about 5^{th} to about 23^{rd} week of pregnancy, more preferred from about 11^{th} to about 15^{th} week of pregnancy, increases the quality of the cells, since they can be differentiated into various cell types, thus improving the tissue quality of the engineered replacements.

Furthermore, the inventors have found that the fetal cells isolated according to step (a) of the above-defined inventive method may be stored frozen, preferably by cryopreservation, before cultivated *in vitro* (e.g. seeded and expanded and further cultivated) for forming the desired tissue replacement. Even more surprisingly, tissue replacements engineered by the use of cryopreserved cells show hardly any difference to replacements formed directly from fresh fetal cells. Preferably, the fetal cells which are to be stored frozen are expanded before cryopreservation. Cryopreservation may conveniently be carried out using conventional medium containing DMSO or comparable compounds such as glycerol.

This preferred embodiment of the present invention enables to store the respective cells for a desired period of time until needed for later applications. Thus, it is possible to build up a prenatal cell library for later, postnatal autologous or allogenic (for example for family members or other genetic relatives) applications.

As mentioned before, the method of the present invention is particular useful for the production of cardiovascular structures for replacing dysfunctional or malfunctional tissues, e.g. in patients suffering from congenital heart diseases.

Preferred cardiovascular structures has replacements of the present invention are heart valves, blood vessels or parts thereof such as patches or heart valves leaflets.

Preferably, the tissue placement is a human tissue replacement.

When cultivating the scaffold in order to develop the mammalian tissue replacement, the cultivation may be under static or dynamic conditions. Dynamic conditions are especially useful for the production of cardiovascular structures as disclosed in EP-A-1 077 072. The cultivation is preferably carried out in a suitable bioreactor such as corresponding devices disclosed in EP-A-1 077 072 or WO-A-2004/10112.

Therefore, especially with respect to the production of cardiovascular structures such as heart valves, blood vessels or parts thereof, the novel approach according to the present invention comprises preferably the following steps:
- harvesting prenatal tissue containing non-embryoblastic fetal cells (e.g. chorionic villi, amniotic fluid, maternal blood or maternal tissue), e.g. by well-established standard methods for isolation at an early stage of pregnancy, preferably between 11^{th} and 15^{th} week of gestation;
- isolation of various fetal cells each of substantially pure type, e.g. about 5 to 30 mg chorionic villi obtained from prenatal sampling by (e.g. tryptic, collagenase) digestion of the villi, preferably in two steps, or from amniotic fluid (e.g. 2,5 to 20ml) or, alternatively, from maternal blood or tissue;
- expansion of isolated fetal cells which may also be used for prenatal diagnostics in order to evaluate the phenotype and the quality of the obtained cells;
- depending on the needs, the cells may be
   - stored frozen (e.g. cryopreserved) for later application; or
   - directly expanded into high cell numbers for the preparation of tissue-engineered living autologous replacements;
- seeding of those cells having an extracellular matrix-forming capacity onto biodegradable three-dimensional matrices (scaffolds) (having the desired shape or form, e.g. a heart valve, vessel or patch);
- culturing in an *in vivo* bioreactor, e.g. under dynamic/static conditions, preferably as disclosed in EP-A-1 077 72;
- endotheliasation of the surfaces of the obtained constructs; and
- explantation of the ready-to-use tissue replacement from the bioreactor

It is clear to a person skilled in the art that not necessarily all of the above-mentioned steps must be carried out. In particular, the steps may overlap and/or one or more steps may be omitted (for example in case that cells are directly used for the fabrication of a replacement, the step of cryopreservation of the cells is omitted). In the case of the production of cardiovascular structures, myofibroblast or fibroblast and/or their progenitor cells are preferably used for building up the extracellular matrix necessary for the formation of a functional connective tissue structure. Further, it is clear for a person skilled in the art that other cells having the capacity to produce an extracellular matrix may be employed. For optimal function of the engineered cardiovascular structure it is necessary to provide the tissue replacement with a surface having antithrombogenic properties. Preferably, endothelial cells are used for this purpose (so-called "endothelialisation"). This process may also be carried out using endothelial progenitor cells or other cells with endothelial or antithrombogenic properties. For the preparation of the tissue replacement common cell media such as DMEM, EGM^{®}-2 etc. may be used which can be supplemented with one or more of the following components: Vascular Endothelial Growth Factor (VEGF), human Fibroblasts Growth Factor (hFGF), human recombinant long-Insulin-like Growth Factor-1 (R3-IGF), human Epidermal Growth Factor (hEGF), gentamycin and amphotericin (GA-1000), hydrocortisone, heparin, ascorbic acid and fetal bovine serum. After a suitably period of time, e.g. four days, attached cells are reseeded and may be used for further cultivation. For cryopreservation, cell medium containing DMSO or similar components may be used.

The method according to the present invention provides several advantages, including:
- isolation of substantially pure cell populations containing all cell types necessary for the production of an engineered tissue that most closely resembles the native counterpart;
- the fetal cells can be harvested from prenatal tissues routinely used for prenatal diagnostics and can be employed as a cell source for tissue engineering such that the same biopsy can be used for tissued engineering as well as for diagnostic purposes without sacrificing any intact donor tissue;
- the tissue replacements according to the present invention are based on isolated prenatal non-embryoblastic fetal cells such that the final implant is ready at or shortly after birth for repair of congenital malformations;
- cells isolated in an early stage of development such as 11^{th} to 15^{th} week of pregnancy provides an extremely high quality of engineered tissue replacements;
- since it has turned out that the isolated fetal cell types may be cryopreserved before using them for the fabrication of the actual tissue placement, it is feasible to build up libraries of prenatal harvested cells for later application (autologous or allogenic implants, e.g. for familiy members or other genetic relatives).

The present inventors have found out for the first time that it is possible to produce mammalian tissue replacements using fetal cells isolated from maternal blood and/or maternal tissue. Therefore, according to a second aspect, the present invention relates generally to a method for producing a mammalian tissue replacement comprising the steps of:
(I) isolating fetal cells from maternal blood and/or tissue *in vitro;* and
(II) cultivating the fetal cells obtained in step (I) under conditions allowing the development of the tissue replacement.

With respect to preferred embodiments of fetal cells, the stage of the maternal blood and/or tissue (week of pregnancy), isolation of cells (in particular FACS, MACS^{®} etc.) cultivating conditions, bioreactors, and preferred tissue replacements that may be produced, it is expressively referred to the description outlined above with the respect to the first aspect of the present invention.

Therefore, according to a third aspect, the present invention generally discloses the use of maternal blood and/or tissue for mammalian tissue engineering *in vitro.*

In particular, the maternal blood and/or tissue functions as a source of non-embryoblastic fetal cells. Preferably, the maternal blood and/or tissue is at the stage of about 5^{th} to about 24^{th}, preferably about 11^{th} to about 15^{th}, week of pregnancy. However, according to this aspect of the present invention, the time point of cell harvest may be chosen according to the requirements of the individual case, i.e. at any time point allowing to obtain fetal cells from the maternal blood or tissue. For example, it is also possible to isolate the corresponding fetal cells form the maternal blood or tissue even after birth. It is especially preferred that the maternal blood and/or tissue is used for the production of a cardiovascular structure, such as a heart valve, blood vessel or part thereof. More preferred, the engineered tissue is a human tissue.

According to a fourth aspect, the present invention provides a method for the replacement of a non-functional or malfunctional tissue in mammalian patient comprising the steps of:
(A) obtaining material containing non-embryoblastic fetal cells;
(B) producing a functional tissue placement *in vitro* by one or more of the above-defined methods of the invention, wherein the fetal cells are isolated from the material obtained in step (A); and
(C) implanting the tissue replacement into the patient.

As already indicated above, the mammalian tissue replacements produced by the above-described methods are especially useful for the treatment of congenital diseases. Therefore, it is preferred that the material containing non-embryoblastic fetal cells is obtained from the non-embryoblastic part of the fetus which develops to the mammalian patient to be treated. This means that an autologous tissue replacement is produced according to step (B). Alternatively, it is also possible that the non-embryoblastic fetal progenitor cells are used to produce a tissue replacement for a genetic relative. A "Genetic relative" of the mammalian patient is a family member or any other individual displaying cell-surface antigens similar to that of the patient in question, e.g. in the case of human patients, the genetic relative shows a similar HLA typisation.

As already described above, the material used for obtaining non-embryoblastic fetal cells may be selected from maternal blood, maternal tissue amniotic fluid and/or chorionic villi. More preferably, the material is obtained at an early stage of pregnancy, in particular at the stage of about 5^{th} to about 23^{rd}, more preferred about 11^{th} to about 15^{th}, week of pregnancy.

Preferably, the patient to be treated according to the inventive method is a human being, more preferably a new born child. As before, the method of the present invention is especially useful for the treatment of congenital cardiovascular diseases where the non-functional or malfunctional tissue is a cardiovascular structure such as a heart valve, blood vessel or any part of such structures (e.g. heart valve leaflets).

The figures show:
- Fig. 1: shows a photograph of a trileaflet heart valve engineered from non-embryoblastic fetal cells derived from chorionic villi.
- Fig. 2: shows photographs of histological analyses of tissue-engineered heart valves based on chorionic villi-derived cells. (A, B) Haematoxyline and eosine (H&E) staining showing layered tissue formation. (C) Trichrom-Masson staining demonstrates excellent production of extracellular matrix (staining is specific for collagen).
- Fig. 3: shows a graphical representation of the results of biochemical analyses of heart valve leaflets produced from chorionic villi-derived prenatal cells. Surprisingly, both cryopreserved and fresh cells show a similar content of extracellular matrix components as well as numbers of alive cells.
- Fig. 4: shows a graphical representation of the mechanical profile of a tissue-engineered heart valve leaflet produced from chorionic-villi-derived cells.

The present invention is further illustrated by the following non-limiting examples.

### EXAMPLES

### Example 1: Production of a trileaflet heart valve from fetal cells isolated from chorionic villi

### Isolation of non-embryoblastic fetal cells

Non-embryoblastic fetal cells (10 to 30 mg) were obtained from routinely prepared chorionic villus samples in the 11^{th} week of pregnancy. Most of the tissues were directly used for prenatal diagnostics. Using 5 mg of chorionic villi sample were isolated by digestion of the obtained tissue. Briefly, the chorionic villi were washed with serum free medium and transferred to a centrifugation tube. Tissue was completly covered with collagenase and incubated at 37 °C. During incubation the tube was shaken every 15 min. After 60 min cells were centrifuged and the supernatant discarded carefully. Cells were suspended in trypsin and incubated for 10 min at 37°C. Afterwards, cells were centrifuged again. After discarding the supernatant the cells were resuspended. From this cell suspension different types of cells could be sorted by magnetic beads using specific antibodies for surface antigens of progenitor cells. Thus, a magnetic beads-coupled antibody against CD133 was used for separating endothelial progenitor cells which were thus trapped on a magnetic column, whereas myoblast/fibroblast progenitors were collected in the flow-through. Then, CD133-positive cells were eluted after de-magnetisation of the column and cultured under conditions specific for endothelial cells, whereas the progenitor cells in the flow-through were differentiated into myoblasts/fibroblasts.

### Expansion of isolated fetal cells

Cells were cultured in specific medium (EGM^{®}-2 supplied by Cambrex Corp., East Rutherford, NJ, USA) containing supplements (vascular endothelial growth factor (VEGF), human fibroblasts growth factor (hFGF), human recombinant long-insulin-like growth factor-1 (R3-IGF), human epidermal growth factor (hEGF), gentamycin and amphotericin (GA-1000), hydrocortisone, heparin, ascorbic acid) and 20% fetal bovine serum.

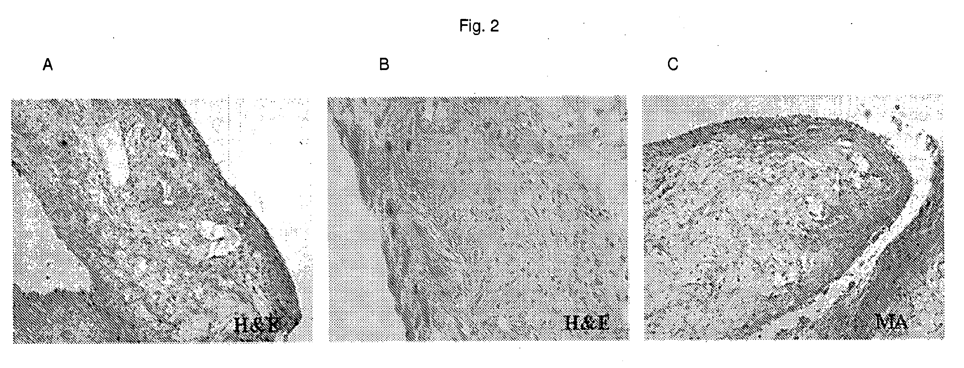

Surprisingly, already after 24 hours cells were attached to the culture dishes and started to proliferate (Fig. 2). Already after 8 days, the cells reached confluency under these culture conditions. Cells were detached by trypsin and half of the cells were cryopreserved using cell medium containing 10% DMSO. The other part was expanded in the following several days into high amounts necessary for the cardiovascular tissue engineering approach. Cryopreserved cells were thawed. When testing the vitality of cells by tryptan-blue staining, surprisingly, the vitality was about 70%. Cells were cultured, expanded and characterised. Non-cryopreserved cells were analysed in parallel. No differences regarding the phenotypes could be detected.

### Seeding of isolated fetal cells onto 3-D matrices and culture

The expanded cells were seeded onto 3-D matrices for the production of tissue replacements. The feasibility of the method according to the present invention was demonstrated by engineering a living, autologous heart valve. Thus, the isolated cells (group 1= cryopreserved cells, group 2= non-cryopreserved cells) characterized as fibroblasts and/or myofibroblasts were seeded onto biodegradable trileaflet heart valve scaffolds (3.5 x 10⁶ cm²) and cultured in an *in vitro* bioreactor. Additionally, a part of the heart valves were exposed to cyclic strain. After 21 weeks heart valves were endothelialised and cultivated for additional 7 days. Thereafter, heart valves were explanted from the bioreactor and the neo-tissue was analysed.

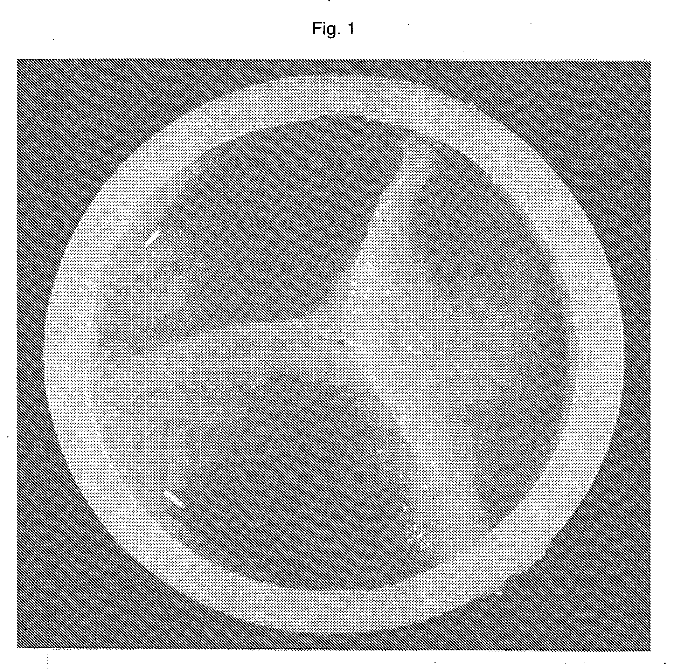

An example of the produced trileaflet heart valves is shown in Fig. 1.

### Analysis of produced heart valves

Analysis included histology, immunohistochemistry, scanning electron microscopy, detection of extracellular matrix production, amount of cells and mechanical testing.

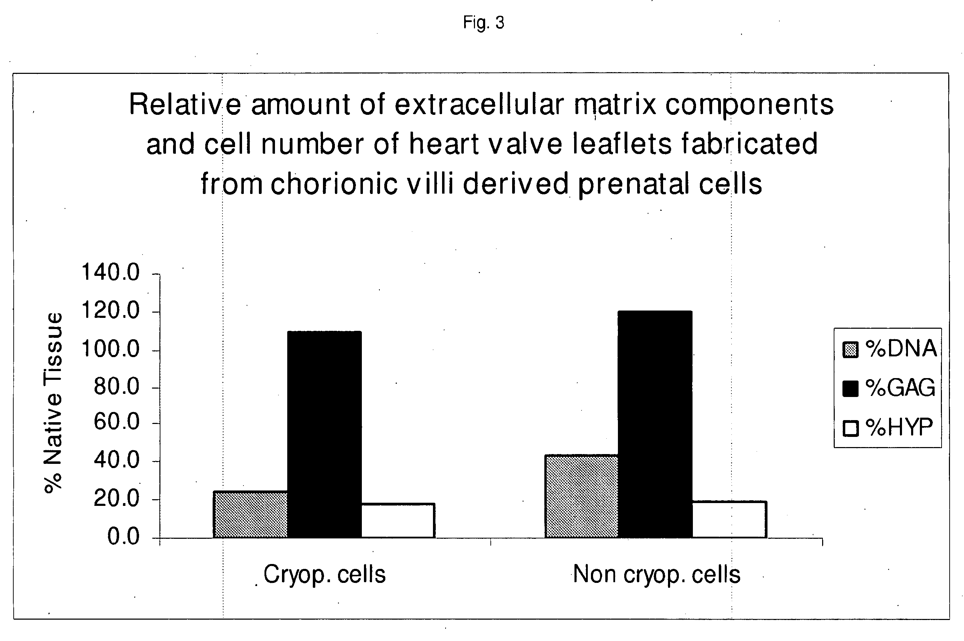

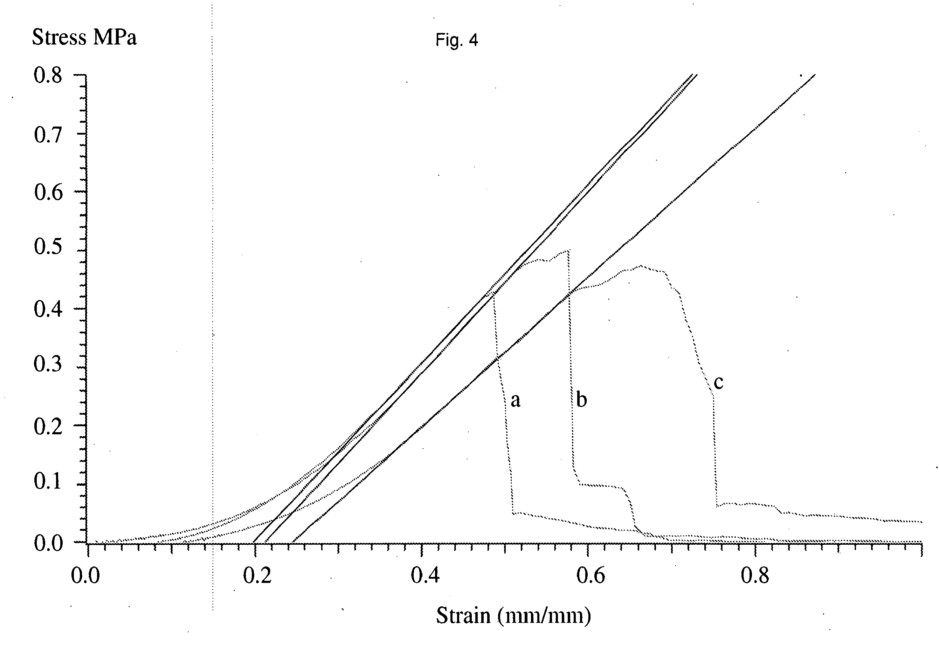

Fresh and cryopreserved placental mesenchymal cells expressed vimentin, desmin and partly alpha-SMA. Surprisingly, neo-tissues demonstrated layered tissue formation and excellent production of extracellular matrix up to native values (glycosaminocglycans (GAG) up to 100%, 4-hydroxyproline (HYP) up to 30%, DNA up to 60%) in both groups (see Fig. 3). Expression of Ki-67 confirmed proliferation of cells in all parts of the neo-tissues. SEM showed excellent cell-ingrowth into the polymer and smooth surfaces. Mechanical testing approximated profiles of native heart valve leaflet tissues (see Fig. 4). When compared to commonly used cells for cardiovascular tissue engineering, chorionic villi-derived prenatal cells are especially suitable for the production of cardiovascular replacements by showing surprisingly better characteristics than replacements produced from commonly used cells. Fig. 2 demonstrates the histology of the neo-tissue. Fig. 4 summarises the biochemical data of the produced heart valves. An example of the mechanical profile of one leaflet is given in Fig. 5. The leaflet produced according to the present invention showed excellent mechanical properties indicated by a Young's modulus of 0.722 ± 0.073 MPa, a tensile strength of 0.250 ± 0.013 MPa, and a strain at break of 0.576 ± 0.089 mm/mm.

### Example 2: Production of a trileaflet heart valve from fetal cells isolated from amniotic fluid

A similar approach was performed using fetal cells isolated from 2.5 ml amniotic fluid obtained in the 15^{th} week of pregnancy, whereas the remainder of the sample was used for prenatal diagnostics. Briefly, the fluid was centrifuged and cells isolated by using magnetic beads for endothelial progenitor cells. The positive coated cells as well as the negative cells were cultured under the culture conditions mentioned above in Example 1 and the steps for the fabrication of the cardiovascular replacement were followed.

Under these culture conditions both types of cells showed excellent proliferation capacity and expressed all typical markers for myofibroblast and/or fibroblast-like cells and endothelial cells and/or endothelial progenitor cells, respectively. This result is highly surprising, since the apoptotic potential of amniotic fluid-derived cells is not fully understood today. Again, surprisingly living, autologous heart valves were generated from prenatal amniotic fluid-derived progenitor cells as amniotic fluid-derived cells formed living cardiovascular tissues covered with functional endothelia.

## Claims

1. A method for the in vitro production of a mammalian tissue replacement comprising the steps of:
(a) preparing one or more substantially pure population(s) of isolated non-embryoblastic fetal CD133-negative cells of one ore more type(s) *in vitro* by cell sorting using an antibody/antibodies directed against the cell type-specific CD 133 surface antigen, wherein the cell type(s) has/have the capacity of forming the native tissue corresponding to the replacement; and
(b) cultivating the fetal cells obtained in step (a) under conditions allowing the development of the tissue replacement,
wherein the fetal cells are fibroblasts and their progenitors,
and wherein step (b) comprises the steps of:
(1) seeding the fetal cells having an extracellular matrix-forming capacity which are fibroblasts or progenitor cells thereof onto a three-dimensional scaffold;
(2) cultivating the scaffold under conditions allowing the development of the tissue replacement until a connective tissue structure has been formed;
(3) seeding fetal cells having antithrombogenic characteristics onto the scaffold containing the connective tissue structure; and
(4) further cultivating the scaffold until at least a monolayer of the fetal cells having antithrombogenic characteristics has been formed on the connective tissue structure.

2. The method of claim 1 wherein the fetal cells include myofibroblast cells.

3. The method according to any one of claims 1 to 2 wherein the fetal cells are isolated from maternal blood, maternal tissue, amniotic fluid, and/or chorionic villi.

4. The method of claim 3 wherein the fetal cells are isolated from chorionic villi at the stage of 5th to 23rd, preferably 11th to 15th, week of pregnancy.

5. The method according to any one of claims 1 to 3 wherein step (a) is performed by flow cytometry, preferably fluorescence-activated cell sorting and/or magnetic cell sorting.

6. The method according to any one of claims 1 to 5 wherein the fetal cells obtained from step (a) are stored frozen before performing step (b).

7. The method of claim 6 wherein the fetal cells are expanded before being stored frozen.

8. The method according to any one of claims 1 to 7 wherein the tissue replacement is a cardiovascular structure.

9. The method of claim 8 wherein the cardiovascular structure is a heart valve, blood vessel or part thereof.

10. The method according to any one of claims 1 to 9 wherein the replacement is a human tissue replacement.

## Patentansprüche

1. Verfahren zur in vitro Herstellung eines Gewebe-Ersatzes für ein Säugetier, aufweisend die folgenden Schritte:
(a) Bereitstellung einer oder mehrerer im Wesentlichen reinen Population(en) von isolierten nicht-embryoblastischen fötalen CD133-negativen Zellen eines Typs oder mehrerer Typen *in vitro* mittels Zellsortierung, unter Verwendung eines Antikörpers / mehrerer Antikörper, welche(r) gegen das zelltyp-spezifische CD133 Oberflächenantigen gerichtet ist/sind, wobei der/die Zelltyp(en) die Fähigkeit hat/haben, das dem Gewebeersatz entsprechende arteigene Gewebe zu bilden; und
(b) Züchten der in Schritt (a) gewonnenen fötalen Zellen, unter Bedingungen, welche die Bildung des Gewebeersatzes erlauben;
wobei die fötalen Zellen Fibroblasten und deren Vorläuferzellen sind;
und wobei Schritt (b) die folgenden Schritte aufweist:
(1) Ansiedeln der fötalen Zellen, welche die Fähigkeit haben, eine extrazelluläre Matrix zu bilden, auf einen dreidimensionalen Träger; wobei diese Zellen Fibroblasten oder deren Vorläuferzellen sind;
(2) Züchten des Trägers unter Bedingungen, welche die Bildung des Gewebeersatzes erlaubt, bis eine Bindegewebsstruktur gebildet ist;
(3) Ansiedeln der fötalen Zellen, welche antithrombogene Eigenschaften haben, auf den Träger, welcher die Bindegewebsstruktur aufweist; und
(4) weitere Züchtung des Trägers, bis auf der Bindegewebsstruktur wenigstens eine Einzelschicht von fötalen Zellen, welche antithrombogene Eigenschaften haben, gebildet ist.

2. Verfahren nach Anspruch 1, wobei die fötalen Zellen unter anderem Myofibroblasten-Zellen sind.

3. Verfahren nach einem der Ansprüche 1 oder 2, wobei die fötalen Zellen aus mütterlichem Blut, mütterlichem Gewebe, Fruchtwasser, und/oder Chorionzotten isoliert werden.

4. Verfahren nach Anspruch 3, wobei die fötalen Zellen im Zeitraum der 5.-23., vorzugsweise der 11.-15. Schwangerschaftswoche aus Chorionzotten isoliert werden.

5. Verfahren nach einem der Ansprüche 1-3, wobei Schritt (a) mittels Durchflusszytometrie, vorzugsweise mittels fluoreszenzaktivierter Zellsortierung und/oder magnetischer Zellsortierung durchgeführt wird.

6. Verfahren nach einem der Ansprüche 1-5, wobei die aus Schritt (a) gewonnenen fötalen Zellen gefroren gelagert werden, bevor Schritt (b) ausgeführt wird.

7. Verfahren nach Anspruch 6, wobei die fötalen Zellen expandiert werden, bevor sie gefroren gelagert werden.

8. Verfahren nach einem der Ansprüche 1-7, wobei der Gewebeersatz eine kardiovaskuläre Struktur ist.

9. Verfahren nach Anspruch 8, wobei die kardiovaskuläre Struktur eine Herzklappe, ein Blutgefäss oder ein Teil davon ist.

10. Verfahren nach einem der Ansprüche 1-9, wobei der Ersatz ein menschlicher Gewebeersatz ist.

## Revendications

1. Procédé pour la production in vitro d'un remplacement de tissu de mammifère comprenant les étapes consistant à :
(a) préparer une ou plusieurs population(s) sensiblement pure(s) de cellules CD133-négatives foetales non embryoblastiques d'un ou plusieurs type(s) *in vitro* par tri cellulaire en utilisant un/des anticorps dirigé(s) contre l'antigène de surface CD133 spécifique à un type de cellules, où le(s) type(s) de cellules a/ont la capacité de formation du tissu natif correspondant au remplacement ; et
(a) cultiver les cellules foetales, obtenues dans l'étape (b) dans des conditions permettant le développement du remplacement de tissu,
dans lequel les cellules foetales sont des fibroblastes et leurs progéniteurs,
et dans lequel l'étape (b) comprend les étapes consistant à :
(1) inoculer les cellules foetales ayant une capacité de formation de matrice extracellulaire qui sont des fibroblastes ou des cellules progénitrices de ceux-ci sur un échafaudage tridimensionnel ;
(2) cultiver l'échafaudage dans des conditions permettant le développement du remplacement de tissu jusqu'à ce qu'une structure de tissu conjonctif se soit formée ;
(3) inoculer des cellules foetales ayant des caractéristiques antithrombogéniques sur l'échafaudage contenant la structure de tissu conjonctif ; et
(4) cultiver plus avant l'échafaudage jusqu'à ce qu'au moins une monocouche des cellules foetales ayant des caractéristiques antithrombogéniques se soit formée sur la structure de tissu conjonctif.

2. Procédé de la revendication 1 dans lequel les cellules foetales comprennent des cellules myofibroblastiques.

3. Procédé selon l'une quelconque des revendications 1 à 2 dans lequel les cellules foetales sont isolées à partir de sang maternel, tissu maternel, fluide amniotique, et/ou villosités choriales.

4. Procédé de la revendication 3 dans lequel les cellules foetales sont isolées à partir de villosités choriales au stade de la 5^{ème} à la 23^{ème}, de préférence de la 11^{ème} à la 15^{ème} semaine de grossesse.

5. Procédé selon l'une quelconque des revendications 1 à 3 dans lequel l'étape (a) est effectuée par cytométrie en flux, de préférence par tri de cellules activé par fluorescence et/ou tri de cellules magnétique.

6. Procédé selon l'une quelconque des revendications 1 à 5 dans lequel les cellules foetales obtenues à partir de l'étape (a) sont stockées à l'état congelé avant d'exécuter l'étape (b).

7. Procédé selon la revendication 6 dans lequel les cellules foetales sont expansées avant d'être stockées à l'état congelé.

8. Procédé selon l'une quelconque des revendications 1 à 7 dans lequel le remplacement de tissu est une structure cardiovasculaire.

9. Procédé de la revendication 8 dans lequel la structure cardiovasculaire est une valvule cardiaque, un vaisseau sanguin ou une partie de ceux-ci.

10. Procédé selon l'une quelconque des revendications 1 à 9 dans lequel le remplacement est un remplacement de tissu humain.
